# EUROPEAN PATENT APPLICATION

(11) **EP 4 707 272 A1**
(43) Date of publication of application: **11.03.2026**
(21) Application number: 25197152.9
(22) Date of filing: 20.08.2025
(51) Int. Cl.: C07C 309/73, H01M 10/0567

(54) **METHOD OF PREPARING BENZENESULFONYL COMPOUND, AND ELECTROLYTE FOR A SECONDARY BATTERY OR A LITHIUM-ION BATTERY COMPRISING THE BENZENESULFONYL COMPOUND**

(30) Priority: 04.09.2024 KR 20240119927
(71) Applicant: SK Innovation Co., Ltd., Seoul 03188 (KR)
(72) Inventor: JIN, Seong Ho, Daejeon 34124 (KR); KIM, Myoung Lae, Daejeon 34124 (KR)
(74) Representative: Prüfer & Partner mbB Patentanwälte · Rechtsanwälte

(57) **Abstract**

Provided is a method of preparing a benzenesulfonyl compound which is performed in the presence of a phase transfer catalyst and an alkali metal hydroxide, and the method has an excellent reaction rate even under mild reaction conditions and may produce the benzenesulfonyl compound in a high yield.

## Description

### TECHNICAL FIELD

The present disclosure relates to a method of preparing a benzenesulfonyl compound. The present disclosure further relates a benzenesulfonyl compound obtained by such method, and the use of a thus obtained benzenesulfonyl compound. The present disclosure also relates to an electrolyte for a secondary battery or a lithium-ion battery comprising the benzenesulfonyl compound.

### BACKGROUND

A benzenesulfonyl compound is known to form a uniform SEI layer on the surface of an electrode and provide high battery stability when it is applied as an electrolytic solution additive. Even though studies of benzenesulfonyl compounds with various structures have been conducted which indicate certain effects as an electrolytic solution additive, studies of synthetic methods thereof are rare and insufficient.

Currently, since the synthesis of the benzenesulfonyl compound has several technical difficulties, it increases production costs during mass production and acts as a factor limiting commercial applicability. For example, upscaling to mass production is difficult due to the use of expensive catalysts, and when the catalysts are intended to be changed to those which can be more easily commercialized, a multi-stage process is involved or a reaction rate becomes slower, the amount of starting materials is significantly increased with respect to the products and the yield is decreased, thereby reducing productivity.

### [Related Art Documents]

### [Non-Patent Literature]

(Non-Patent Document 1) Bioorganic & Medicinal Chemistry Letters (2022), 75, 128956

### SUMMARY

An embodiment of the present invention is directed to providing a method of preparing a benzenesulfonyl compound in a significantly improved yield and excellent productivity, even by a simple process.

In one general aspect, a method of preparing a benzenesulfonyl compound includes: preparing a benzenesulfonyl compound represented by the following Chemical Formula 1 by reacting a compound represented by the following Chemical Formula 2 and an n-hydric alcohol in the presence of a phase transfer catalyst and an alkali metal hydroxide aqueous solution: 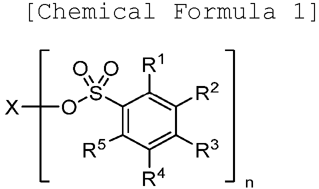 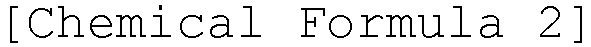 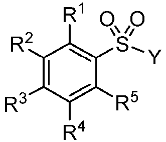 wherein
R¹ to R⁵ are independently of one another hydrogen, a halogen, C1-C7 alkoxy, or C1-C7 alkyl;
X is a residue derived from the n-hydric alcohol;
n is an integer of 1 to 4; and
Y is a halogen.

In Chemical Formula 1, in an exemplary embodiment X may be a residue derived from the a poly-hydric alcohol, preferably is a residue derived from ethylene glycol or glycerol.

The alkali metal hydroxide aqueous solution may include 30 to 80 wt%, preferably 30 to 70 wt%, more preferably 40 to 70 wt%, and even more preferably 50 to 70 wt% of the alkali metal hydroxide.

The alkali metal hydroxide aqueous solution may contain sodium hydroxide and/or potassium hydroxide.

The phase transfer catalyst may be an ammonium-based catalyst.

The compound represented by Chemical Formula 2 may be used at 0.3n to 1n mol with respect to 1 mol of the n-hydric alcohol, in which n may be an integer of 1 to 4.

The compound represented by Chemical Formula 2 may be used at 0.3n to 0.8n mol with respect to 1 mol of the n-hydric alcohol, in which n may be an integer of 1 to 4.

The alkali metal hydroxide may be used at 1 mol to 5 mol with respect to 1 mol of the compound of Chemical Formula 2.

The alkali metal hydroxide and the phase transfer catalyst may be used at a molar ratio of 1:0.001 to 0.1, preferably at a molar ratio of 1:0.005 to 0.05.

In the method of preparing a benzenesulfonyl compound according to an exemplary embodiment may be performed under a temperature condition of 20 to 40°C for 1 to 10 hours.

In an embodiment, the internal temperature of the reaction mixture may be maintained at less than 30°C.

In an embodiment, the reaction mixture may be continuously stirred.

In the method of preparing a benzenesulfonyl compound according to an exemplary embodiment, a yield of the benzenesulfonyl compound may be 60% or more.

In a further aspect of the present disclosure, there is provided a benzenesulfonyl compound of the Chemical Formula 1 which is obtained by the method as disclosed herein.

In an aspect, a benzenesulfonyl compound is provided in which the benzenesulfonyl compound of Chemical Formula 1 has one of the following chemical structures:

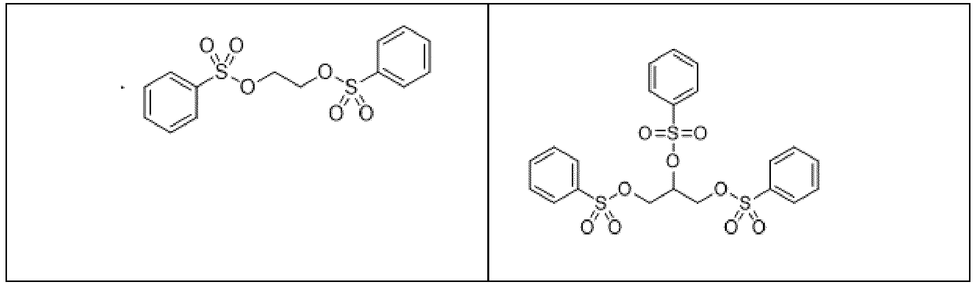

.

The benzenesulfonyl compound may be used as an additive for an electrolyte of a secondary battery or a lithium-ion battery.

According to a further aspect, in a first step a benzenesulfonyl compound of the Chemical Formula 1 is obtained by the method as disclosed herein, and as a second step the prepared benzenesulfonyl compound is used as an additive for an electrolyte of a secondary battery or a lithium-ion battery. By such a process, an electrolyte of a secondary battery or a lithium-ion battery may be prepared.

In a further aspect, the present disclosure relates to an electrolyte for a secondary battery or a lithium-ion battery, wherein the electrolyte comprises the benzenesulfonyl compound of the [Chemical Formula 1] disclosed herein. The electrolyte may contain components that are conventional and typical for an electrolyte for a secondary battery or a lithium-ion battery, such as lithium salts, solvents, and/or additives other than benzenesulfonyl compound of the [Chemical Formula 1] disclosed herein.

Other features and aspects will be apparent from the following detailed description, the drawings, and the claims.

### DETAILED DESCRIPTION OF EMBODIMENTS

In the present specification, unless otherwise defined, all technical terms and scientific terms have the same meanings as those commonly understood by a person skilled in the art to which the present invention pertains. The terms used herein are only for effectively describing a certain specific example and are not intended to limit the present invention.

The singular form used in the present specification may be intended to also include a plural form, unless otherwise indicated in the context.

Throughout the present specification, unless otherwise particularly stated, each of the terms "include", "comprise", "equipped", "contain", or "have" does not mean the exclusion of any other constituent element, but mean further inclusion of other constituent elements, and elements, materials, or processes which are not further listed are not excluded.

The numerical range used in the present specification includes all values within the range including the lower limit and the upper limit, increments logically derived from the form and spanning of a defined range, all double limited values, and all possible combinations of the upper limit and the lower limit in the numerical range defined in different forms. Unless otherwise defined in the present specification, values which may be outside a numerical range due to experimental error or rounding off of a value are also included in the defined numerical range.

Unless otherwise particularly defined in the present specification, "about" may be considered as a value within 30%, 25%, 20%, 15%, 10%, or 5% of a stated value.

Hereinafter, the present disclosure will be described in detail. However, it is only illustrative, and the present disclosure is not limited to the specific exemplary embodiment which is illustratively described.

The term "halogen" in the present specification may refer to fluorine (F), chlorine (Cl), bromine (Br), or iodine (I) atom.

The term "alkyl" in the present specification is an organic radical derived from an aliphatic hydrocarbon by removal of one hydrogen and may include both a straight chain and branched chain forms. The alkyl may have 1 to 7 carbon atoms, specifically 1 to 5 carbon atoms, specifically 1 to 4 carbon atoms, or specifically 1 to 3 carbon atoms. The alkyl includes, as an example, methyl, ethyl, propyl, isopropyl, butyl, isobutyl, t-butyl, pentyl, hexyl, ethylhexyl, and other linear, branched or cyclic alkyl groups, but is not limited thereto.

The term "alkoxy" in the present specification is indicated as *-O-alkyl in which alkyl is as defined above. The alkoxy includes, as an example, methoxy, ethoxy, n-propoxy, isopropoxy, n-butoxy, isobutoxy, t-butoxy, and other linear, branched or cyclic alkoxy groups, but is not limited thereto.

The term "yield" in the present specification is defined as the product of selectivity and conversion. In this context, "yield" refers to the amount of desired product obtained from a chemical reaction, expressed as a percentage of the theoretical maximum.

An exemplary embodiment provides a method of preparing a benzenesulfonyl compound, which may satisfy simplification of a preparation process, yield improvement, and productivity simultaneously.

Specifically, the method of preparing a benzenesulfonyl compound according to an exemplary embodiment may include: preparing a benzenesulfonyl compound represented by the following Chemical Formula 1 by reacting a compound represented by the following Chemical Formula 2 and an n-hydric alcohol in the presence of a phase transfer catalyst and an alkali metal hydroxide aqueous solution: 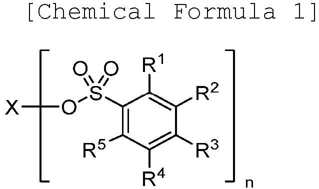 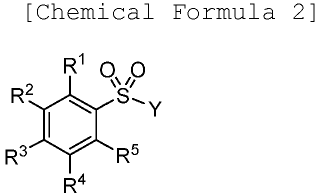 wherein
R¹ to R⁵ are independently of one another hydrogen, a halogen, C1-C7 alkoxy, or C1-C7 alkyl;
X is a residue derived from the n-hydric alcohol;
n is an integer of 1 to 4; and
Y is a halogen.

In a conventional method of preparing a benzenesulfonyl compound, when a base catalyst used under economical considerations, such as an alkali metal hydroxide, is introduced for securing economic feasibility, an excessive amount of catalyst is used, and both a reaction rate and a product yield are reduced. Since the preparation method according to an exemplary embodiment uses a combination of the phase transfer catalyst and the alkali metal hydroxide aqueous solution, it has an excellent reaction rate even under mild reaction conditions and may produce the benzenesulfonyl compound in a high yield.

The alkali metal hydroxide aqueous solution may include 20 to 80 wt%, 30 to 80 wt%, 40 to 80 wt%, 40 to 70 wt%, or 40 to 60 wt% of an alkali metal hydroxide and may further increase the yield. In terms of an increased product yield at an enhanced overall reaction rate even under mild reaction conditions, a content of the alkali metal hydroxide(s) included in the alkali metal hydroxide aqueous solution in a range of 30 to 70 wt% or 50 to 70 wt% may be particularly beneficial.

The alkali metal hydroxide metal may be used at 0.1 mol to 10 mol, 1 mol to 10 mol, 1 mol to 8 mol, 1 mol to 6 mol, 1 mol to 5 mol, 1 mol to 3 mol, 1.2 mol to 3 mol, 1.5 mol to 3 mol, or 1.5 mol to 2 mol with respect to 1 mol of the compound represented by Chemical Formula 2, and when the alkali metal hydroxide aqueous solution within the range of concentration described above is used to satisfy the molar ratio, the reaction rate and the product yield may be higher.

The alkali metal hydroxide and the phase transfer catalyst may be used at a molar ratio of 1:0.001 to 0.1, 1:0.001 to 0.05, 1:0.005 to 0.05, 1:0.005 to 0.03, or 1:0.01 to 0.03.

The alkali metal hydroxide may be sodium hydroxide (NaOH) or potassium hydroxide (KOH), or a combination thereof.

The phase transfer catalyst (PTC) may be an ammonium-based catalyst, and as an example, may be ammonium halide catalysts such as tetramethylammonium bromide, tetramethylammonium chloride, tetraethylammonium bromide, tetrapropylammonium bromide, tetrabutylammonium bromide, tetrabutylammonium chloride, cetyltriammonium bromide, benzyltriethylammonium chloride, and trioctylmethylammonium chloride.

The compound represented by Chemical Formula 2 may be used at 0.2n mol or more, 0.3n mol or more and 2n mol or less, 1.5n mol or less, or 1n mol or less, specifically 0.2n to 2n mol, 0.3n to 1.5n mol, 0.3n to 1.2n mol, 0.3n to 1.0n mol, 0.3n to 0.9n mol, or 0.3n to 0.8n mol, with respect to 1 mol of the n-hydric alcohol, may further increase the yield of the benzenesulfonyl compound, and may include all possible combinations of the upper limit and the lower limit of the numerical range, and n may be an integer of 1 to 4 or an integer of 2 to 4.

The n-hydric alcohol refers to an alcohol having n hydroxyl groups (-OH) and may be monohydric alcohol, dihydric alcohol, trihydric alcohol, or tetrahydric alcohol. As an example, the n-hydric alcohol may be a monohydric alcohol selected from methanol, ethanol, 1-propanol, 2-propanol, 1-butanol, 2-butanol, 1-pentanol, 2-pentanol, 3-pentanol, 1-hexanol, 2-hexanol, 3-hexanol, and other hydroxyl derivates of hydrocarbons comprising one hydroxyl group; a dihydric alcohol selected from ethylene glycol, diethylene glycol, triethylene glycol, 1,2-propanediol, 1,3-propanediol, 1,3-butanediol, 1,4-butanediol, 1,5-pentanediol, 2,2-dimethyl-1,3-propanediol, 1,6-hexanediol, and other hydroxyl derivates of hydrocarbons comprising two hydroxyl groups; a trihydric alcohol such as glycerol and trimethylolpropane; and a tetrahydric alcohol such as pentaerythritol and erythritol, but is not limited thereto.

As an example, the n-hydric alcohol may be a dihydric alcohol, and the compound represented by Chemical Formula 2 may be used at 0.5 to 3 mol, 0.5 to 2 mol, 0.6 to 2.5 mol, 0.6 to 2 mol, 0.6 to 1.8 mol, or 0.6 to 1.6 mol with respect to 1 mol of the dihydric alcohol, and may include all possible combinations of the upper limit and the lower limit of the numerical range.

As an example, the n-hydric alcohol may be a trihydric alcohol, and the compound represented by Chemical Formula 2 may be used at 0.5 to 4 mol, 0.5 to 3 mol, 1 to 3 mol, 1 to 2.5 mol, or 1 to 2 mol with respect to 1 mol of the trihydric alcohol, and may include all possible combinations of the upper limit and the lower limit of the numerical range.

As an example, R¹ to R⁵ may be independently of one another hydrogen, a halogen, C1-C4 alkoxy, or C1-C4 alkyl, and may be hydrogen, fluoro (-F), or methyl.

The preparation method according to an exemplary embodiment has an excellent reaction rate even under mild reaction conditions and may produce the benzenesulfonyl compound in a high yield. As an example, the initial reaction temperature may be below 30°C, preferably below 10°C, and more preferably at about 0°C, and the reaction may be continued under a temperature condition of 20 to 40°C for 1 to 10 hours, 2 to 10 hours, or 3 to 8 hours, and the yield of the benzenesulfonyl compound may be 60% or more, 70% or more, 75% or more, 80% or more, or 90% or more, in which the upper limit may be 99%.

In an embodiment, the benzenesulfonyl compound obtained by the method disclosed in the present disclosure may be used as an electrolyte additive in a secondary battery or a lithium-ion battery.

In a further aspect, the present disclosure provides an electrolyte for a secondary battery or a lithium-ion battery, wherein the electrolyte comprises the benzenesulfonyl compound of the [Chemical Formula 1] disclosed herein. The electrolyte may contain further components suitable or useful for an electrolyte for a secondary battery or a lithium-ion battery, for example selected from the groups consisting of lithium salts, solvents, and/or additives other than said benzenesulfonyl compound. Suitable lithium salts may be selected from the group consisting of, for instance, LiPF₆, LiClO₄, LiBF₄ and LiAsF₆, without being limited thereto; suitable solvents may be selected from the group consisting of, for instance, cyclic carbonates such as propylene carbonate (PC), ethylene carbonate (EC), linear alkyl carbonates such as diethyl carbonate (DEC), diethyl carbonate (DMC), and ethyl methyl carbonate (EMC), and carboxyl esters such as methyl formate (MF), methyl acetate (MA), ethyl acetate (EA), methyl propionate (MP), etc., without being limited thereto; suitable other additives may be selected from the group consisting of, for instance, film-forming additives, conductive additives, flame retardants, etc., without being limited thereto.

Hereinafter, the exemplary embodiments described above will be described in detail through the following examples. However, the following examples are only for description, and do not limit the right scope.

### [Example 1]

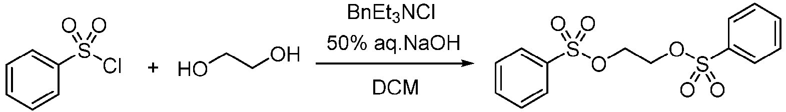

A round bottom flask, to which 0.15 mol of ethylene glycol, 0.005 mol of benzyltriethylammonium chloride (BnEt₃NCl) and 30 mL of dichloromethane (DCM) have been added, was cooled to 0°C, and 0.2 mol of benzenesulfonyl chloride was added under stirring. Subsequently, 0.3 mol of sodium hydroxide was added as 50 wt% of a sodium hydroxide aqueous solution under stirring. During the addition, the internal temperature was maintained at less than 30°C, and after the addition, stirring was further performed at room temperature (25°C) for 5 hours. After the reaction was finished, an organic layer was washed with water twice, concentrated, and recrystallized in methanol to obtain 1,2-bis(benzenesulfonyloxy)ethane (93%).

¹H-NMR (CDCl₃, 500MHz, ppm): 7.87 (4H, m), 7.68 (2H, m), 7.56(4H, m), 4.23(4H, s).

### [Example 2]

The process was performed in the same manner as in Example 1, except that the amount of ethylene glycol used was changed to 0.13 mol.

### [Example 3]

The process was performed in the same manner as in Example 1, except that the amount of ethylene glycol used was changed to 0.1 mol.

### [Example 4]

The process was performed in the same manner as in Example 3, except that the amount of sodium hydroxide used was changed to 0.2 mol.

### [Example 5]

The process was performed in the same manner as in Example 3, except that 30 wt% of a sodium hydroxide aqueous solution was used instead of 50 wt% of a sodium hydroxide aqueous solution.

### [Example 6]

The process was performed in the same manner as in Example 1, except that the amount of benzyltriethylammonium chloride used was changed to 0.002 mol.

### [Example 7]

The process was performed in the same manner as in Example 1, except that the amount of ethylene glycol used was changed to 0.3 mol.

### [Example 8]

The process was performed in the same manner as in Example 1, except that tetrabutylammonium bromide was used instead of benzyltriethylammonium chloride.

### [Example 9]

The process was performed in the same manner as in Example 1, except that 50 wt% of a potassium hydroxide aqueous solution was used instead of 50 wt% of a sodium hydroxide aqueous solution.

### [Comparative Example 1]

The process was performed in the same manner as in Example 4, except that benzyltriethylammonium chloride was not used.

### [Comparative Example 2]

The process was performed in the same manner as in Example 4, except that 0.05 mol of triethylamine was used instead of benzyltriethylammonium chloride.

The molar ratio described in the following Table 1 refers to the molar ratio with respect to 1 mol of benzenesulfonyl chloride.

**[Table 1]**

| | Ethylene glycol | Alkali metal hydroxide aqueous solution | | Catalyst | | Yield (%) |
|---|---|---|---|---|---|---|
| | Molar ratio | Type | Molar ratio | Type | Molar ratio | |
| Example 1 | 0.75 | 50 wt% NaOH | 1.5 | BnEt₃NCl | 0.025 | 93 |
| Example 2 | 0.65 | 50 wt% NaOH | 1.5 | BnEt₃NCl | 0.025 | 90 |
| Example 3 | 0.5 | 50 wt% NaOH | 1.5 | BnEt₃NCl | 0.025 | 81 |
| Example 4 | 0.5 | 50 wt% NaOH | 1.0 | BnEt₃NCl | 0.025 | 73 |
| Example 5 | 0.5 | 30 wt% NaOH | 1.5 | BnEt₃NCl | 0.025 | 65 |
| Example 6 | 0.75 | 50 wt% NaOH | 1.5 | BnEt₃NCl | 0.01 | 88 |
| Example 7 | 1.5 | 50 wt% NaOH | 1.5 | BnEt₃NCl | 0.025 | 95 |
| Example 8 | 0.75 | 50 wt% NaOH | 1.5 | BU₄NBr | 0.025 | 91 |
| Example 9 | 0.75 | 50 wt% KOH | 1.5 | BnEt₃NCl | 0.025 | 92 |
| Comparative Example 1 | 0.5 | 50 wt% NaOH | 1.0 | - | - | 56 |
| Comparative Example 2 | 0.5 | 50 wt% NaOH | 1.0 | Et3N | 0.25 | 59 |

### [Example 10]

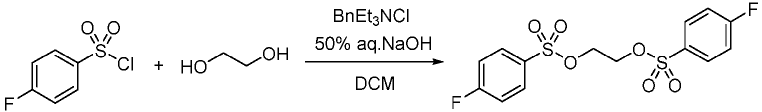

1,2-bis(4-fluorobenzenesulfonyloxy)ethane was obtained (94%) in the same manner as in Example 1, except that 4-fluorobenzenesulfonyl chloride was used instead of benzenesulfonyl chloride.

¹H-NMR (CDCl₃, 500MHz, ppm): 7.89 (4H, m), 7.23 (4H, m), 4.25(4H, s).

### [Example 11]

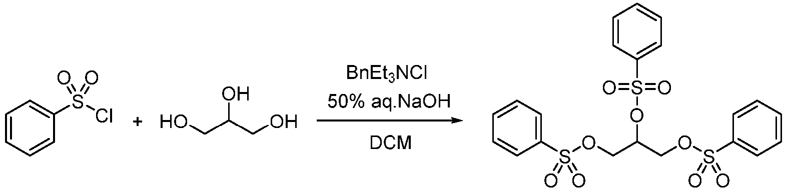

1,2,3-tris (benzenesulfonyloxy)propane was obtained (90%) in the same manner as in Example 1, except that 0.1 mol of glycerol was used instead of 0.15 mol of ethylene glycol.

¹H-NMR (CDCl₃, 500MHz, ppm): 7.82 (6H, m), 7.68(3H, m), 7.55(6H, m), 4.74(1H, p), 4.13(4H, d).

The method of preparing a benzenesulfonyl compound according to an exemplary embodiment may provide a benzenesulfonyl compound in an excellent yield by a simple process. Specifically, since the preparation method according to an exemplary embodiment has an excellent reaction rate even under mild reaction conditions to have a short reaction time, and may produce a benzenesulfonyl compound in a high yield by using economical materials, it has excellent productivity and may be favorable for application to practical industrial sites.

Hereinabove, although the present invention has been described by specified matters and specific exemplary embodiments, they have been provided only for assisting in the entire understanding of the present invention, and the present invention is not limited to the exemplary embodiments.

## Claims

1. A method of preparing a benzenesulfonyl compound, the method comprising:
preparing a benzenesulfonyl compound represented by the following Chemical Formula 1 by reacting a compound represented by the following Chemical Formula 2 and an n-hydric alcohol in the presence of a phase transfer catalyst and an alkali metal hydroxide aqueous solution: 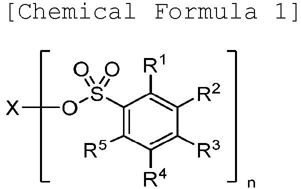 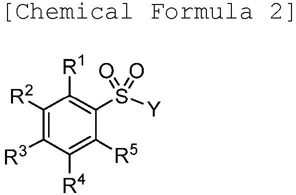 wherein
R¹ to R⁵ are independently of one another hydrogen, a halogen, C1-C7 alkoxy, or C1-C7 alkyl;
X is a residue derived from the n-hydric alcohol;
n is an integer of 1 to 4; and
Y is a halogen.

2. The method of preparing a benzenesulfonyl compound according to claim 1, wherein in Chemical Formula 1, X is a residue derived from the a poly-hydric alcohol, preferably is a residue derived from ethylene glycol or glycerol.

3. The method of preparing a benzenesulfonyl compound according to claim 1 or 2, wherein the alkali metal hydroxide aqueous solution includes 30 to 80 wt%, preferably 30 to 70 wt%, more preferably 40 to 70 wt%, and even more preferably 50 to 70 wt% of the alkali metal hydroxide.

4. The method of preparing a benzenesulfonyl compound according to any one of the preceding claims, wherein the alkali metal hydroxide aqueous solution contains sodium hydroxide and/or potassium hydroxide.

5. The method of preparing a benzenesulfonyl compound according to any one of the preceding claims, wherein the phase transfer catalyst is an ammonium-based catalyst.

6. The method of preparing a benzenesulfonyl compound according to any one of the preceding claims, wherein the compound represented by Chemical Formula 2 is used at 0.3n to 1n mol with respect to 1 mol of the n-hydric alcohol, in which n is an integer of 1 to 4.

7. The method of preparing a benzenesulfonyl compound according to claim 5, wherein the compound represented by Chemical Formula 2 is used at 0.3n to 0.8n mol with respect to 1 mol of the n-hydric alcohol, in which n is an integer of 1 to 4.

8. The method of preparing a benzenesulfonyl compound according to any one of the preceding claims, wherein the alkali metal hydroxide is used at 1 mol to 5 mol with respect to 1 mol of the compound of Chemical Formula 2.

9. The method of preparing a benzenesulfonyl compound according to any one of the preceding claims, wherein the alkali metal hydroxide and the phase transfer catalyst are used at a molar ratio of 1:0.001 to 0.1, preferably at a molar ratio of 1:0.005 to 0.05.

10. The method of preparing a benzenesulfonyl compound according to any one of the preceding claims, wherein the reaction is performed under a temperature condition of 20 to 40°C for 1 to 10 hours.

11. The method of preparing a benzenesulfonyl compound according to any one of the preceding claims, wherein the internal temperature of the reaction mixture is maintained at less than 30°C.

12. The method of preparing a benzenesulfonyl compound according to any one of the preceding claims, wherein the reaction mixture is continuously stirred.

13. The method of preparing a benzenesulfonyl compound according to any one of the preceding claims, wherein a yield of the benzenesulfonyl compound is 60% or more.

14. A benzenesulfonyl compound of the following Chemical Formula 1 obtained by the method according to any one of the preceding claims, 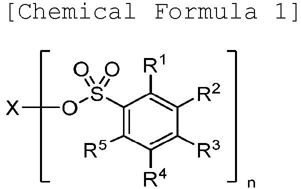 wherein
R¹ to R⁵ are independently of one another hydrogen, a halogen, C1-C7 alkoxy, or C1-C7 alkyl;
X is a residue derived from an n-hydric alcohol; and
n is an integer of 1 to 4.

15. An electrolyte for a secondary battery or a lithium-ion battery, wherein the electrolyte comprises a benzenesulfonyl compound of the following Chemical Formula 1, and optionally further conventional components for an electrolyte for a secondary battery or a lithium-ion battery, selected from the group consisting of lithium salts, solvents, and/or additives other than said benzenesulfonyl compound, 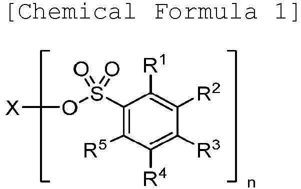 wherein
R¹ to R⁵ are independently of one another hydrogen, a halogen, C1-C7 alkoxy, or C1-C7 alkyl;
X is a residue derived from an n-hydric alcohol; and n is an integer of 1 to 4.
